# EUROPÄISCHE PATENTSCHRIFT

(11) **EP 0 454 105 B1**
(45) Veröffentlichungstag und Bekanntmachung des Hinweises auf die Patenterteilung: **02.11.1995**
(21) Anmeldenummer: 91106631.4
(22) Anmeldetag: 24.04.1991
(51) Int. Cl.: A61F 13/15

(54) **Wegwerfhöschen zur Erziehung**
Disposable training panties
Couche-culotte jetable pour éducation hygiénique

(30) Priorität: 24.04.1990 JP 109641/90; 25.04.1990 JP 109300/90
(43) Veröffentlichungstag der Anmeldung: 30.10.1991
(73) Patentinhaber: UNI-CHARM CORPORATION, Kawanoe-shi Ehime-ken (JP)
(72) Erfinder: Igaue, Takamitsu, Kawanoe-shi, Ehime-ken (JP); Sasaki, Tohru, Kawonoe-shi, Ehime-ken (JP); Kitaoka, Hideaki, Funabashi-shi, Chiba-ken (JP)
(74) Vertreter: Sperling, Rüdiger, Dipl.-Ing.

(56) Entgegenhaltungen:
- GB-A- 2 089 853
- US-A- 3 613 687
- US-A- 4 735 622
- US-A- 4 755 179

## Beschreibung

Die Erfindung betrifft ein Wegwerfhöschen zur Erziehung, mit einer elastisch dehnbaren Hüftöffnung und zwei elastisch dehnbaren Beinöffnungen, das eine flüssigkeitsdurchlässige innere Lage, eine flüssigkeitsundurchlässige äußere Lage und einen zwischen dieser inneren und äußeren Lage liegenden absorbieren Kern umfaßt.

Solche Erziehungshöschen werden verwendet, um Babys zu veranlassen, ohne Widerstreben die Gewohnheit, sich auf eine Windel zu verlassen, aufzugeben, und sie sobald als möglich an ein Leben zu gewöhnen, das keine Windeln erforderlich macht.

Herkömmliche Erziehungshöschen waren im allgemeinen aus Stoff hergestellt und zur mehrfachen Verwendung waschbar ausgelegt.

Dieses Waschen war gewöhnlich unangenehm und darüber hinaus war es ein ernsthaftes Problem, daß oftmals eine gewisse Menge von flüssigen Ausscheidungen aus den Höschen heraussikkerte und den Fußboden in der Wohnung beschmutzte, wenn ein das Höschen tragendes Baby dieses mit Ausscheidungen verunreinigte.

Aus der US-PS 3,613,687 ist eine waschbare, schnelltrocknende Windelhose bekannt, die gemäß Offenbarung einerseites eine hohe Flüssigkeitsrückhaltekraft aufweist, andererseits aber schnell trocknend sein soll. Diese Windelhose besitzt im Hüftbereich ein Baumwollgestrick, das auf einer Raschelmaschine hergestellt worden ist. Im Schrittbereich erstreckt sich auf der Vorder- und Rückseite bis zu einem elastischen Hüftband ein dreilagiger Abschnitt. Die äußere Lage besteht aus einem wiederum auf einer Raschelmaschine hergestellten Gestrick, wohingegen die innere, dem Körper zugekehrte Lage aus einem Baumwolljerseygestrick besteht. Zwischen beiden Lagen ist eine hoch saugfähige Polyestermateriallage angeordnet. Die diesen verschiedenen Lagen beigegebenen Eigenschaften sind ausschließlich auf das Ziel gerichtet, eine waschfähige Windelhose bereitzustellen, die in kürzester Zeit trocknet.
So ist beispielsweise die innere, dem Körper zugewandte Lage nicht deshalb aus vorherrschend hydrophilen Fasern hergestellt, um ein Feuchtigkeitsgefühl zu erzeugen. Vielmehr ist die Struktur ausgelegt, um möglichst unbeschadet Waschprozeduren zu überstehen. Insbesonders wird diese Lage als eine Gazelage beschrieben, sodaß diese Lage fast keine Feuchtigkeit speichert.

Aus der US-PS 4,735,622 ist ein Windelhöschen für Trainingszwecke bekannt, bei dem ein "happy face", d.h. ein lächelndes Gesicht auf dem Vorderteil ca. in Bauchhöhe angebracht ist, das zu einem traurigen Gesicht umwechselt, wenn eine innere Lage aus absorbierendem Material, die am Körper des Kindes anliegt, naß wird.

Der Erfindung liegt die Aufgabe zugrunde, eine verbesserte Wegwerfwindel vorzuschlagen, die so ausgelegt ist, daß sie nicht nur in geeigneter Weise als Erziehungshöschen wirkt dergestalt, daß in dem Fall, wenn ein das Höschen tragendes Baby Ausscheidungen absondert, das von den flüssigen Aus scheidungen benetzte Höschen dem Baby ein unangenehmes Geführt vermittelt, sondern auch verhindert, daß diese flüssigen Ausscheidungen in irgendeiner Menge aus dem Höschen austreten.

Gemäß der vorliegenden Erfindung wird die vorstehend gestellte Aufgabe dadurch gelöst, daß eine vliesartige Lage, die an einem bestimmten Abschnitt der inneren Oberfläche der inneren Lage befestigt ist und die Feuchtigkeitsrückhalteeigenschaften aufweist, die größer sind als derartige Eigenschaften der inneren Lage, um eine Feuchtigkeitsfühlzone (Ffz) zu schaffen, die von dem Träger als unangenehm empfunden wird, sobald eine Ausscheidung stattgefunden hat.

Sondert ein das erfindungsgemäße Höschen tragendes Baby Ausscheidungen ab, so wird dadurch die an der inneren Lage vorgesehene Feuchtigkeitsfühlzone angefeuchtet und in direkten Kontakt mit der Haut des Babys gebracht. Als Folge davon vermittelt die Feuchtigkeitsfühlzone dem Baby ein unangenehmes Gefühl und das Baby spürt, daß es Ausscheidungen abgesondert hat. Somit lässt die Verwendung des Höschens in effektiver Weise das Baby die Gewohnheit annehmen, es anderen mitzuteilen, wenn es Ausscheidungen abgesondert hat.

Zusätzlich zu dieser zweckmässigen Funktion des Erziehungshöschens zeigt ein weiteres Merkmal der vorliegenden Erfindung insofern einen wichtigen Vorteil auf, als der Fußboden in einer Wohnung davor geschützt ist, durch das mögliche Austreten von flüssigen Ausscheidungen verschmutzt zu werden, da die flüssigkeitsundurchlässige äußere Lage in wirksamer Weise das Austreten von flüssigen Ausscheidungen aus dem Höschen verhindert.

Bei dem dem erfindungsgemäßen Aufbau entsprechenden Höschen wirkt die flüssigkeitsundurchlässige äußere Lage blockierend auf die flüssigen Ausscheidungen und verbessert entsprechend den Befeuchtungseffekt der Feuchtigkeitsfühlzonen. Dies wiederum verbessert die zweckmässige Funktion des Erziehungshöschens. Dieses Merkmal ist bei den herkömmlichen, aus Stoff gefertigten Erziehungshöschen nicht gegeben.

### KURZE BESCHREIBUNG DER ZEICHNUNGEN

Fig. 1 zeigt eine isometrische Darstellung einer Ausführungsform des gemäß vorliegender Erfindung aufgebauten Erziehungshöschens;
Fig. 2 ist eine Draufsicht auf das in Fig. 1 dargestellte Höschen in entfaltetem Zustand, die eine mit einer Feuchtigkeitsfühlzone versehene innere Lage zeigt; und
Fig. 3 bis 5 sind Draufsichten auf einen Absorptionskern, der jeweils mit einer Feuchtigkeitsfühlzone in verschiedenen Anordnungen versehen ist.

### BEVORZUGTE AUSFÜHRUNGSFORMEN DER ERFINDUNG

Die vorliegende Erfindung wird anhand von Beispielen unter Bezug auf die beigefügten Zeichnungen erläutert.

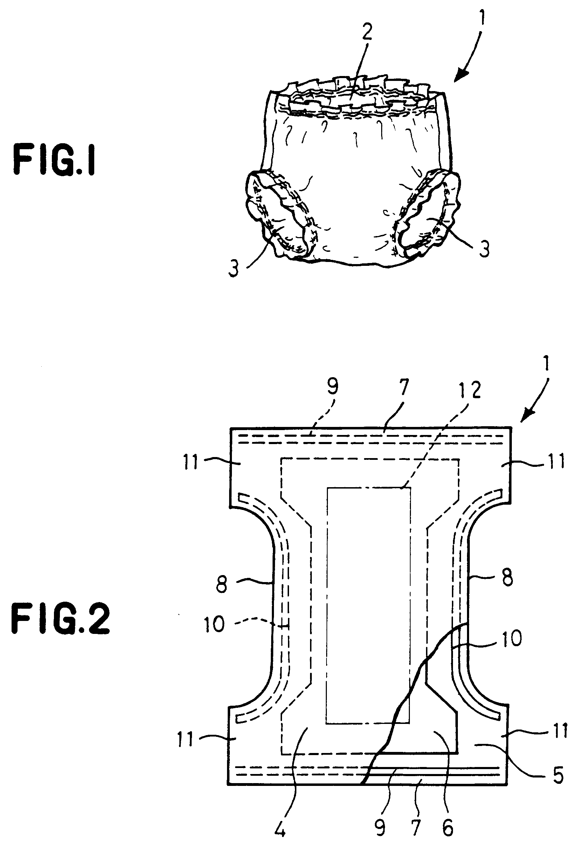

Wie in Fig. 1 und 2 gezeigt, umfaßt ein Höschen 1 eine elastisch dehnbare Hüftöffnung 2 und zwei elastisch dehnbare Beinöffnungen 3. Das Höschen 1 umfaßt im wesentlichen eine flüssigkeitsdurchlässige innere Lage 4, eine flüssigkeitsundurchlässige äußere Lage 5 und eine flüssigkeitsabsorbierenden Kern 6 in Form einer Schicht oder Matte.

Zwischen der inneren und der äußeren Lage 4, 5 sind in gestrecktem Zustand mittels Klebstoffs elastisch dehnbare Elemente 9, 10 entlang Bereichen 7, 8, die die Hüftöffnung bzw. die Beinöffnungen begrenzen, befestigt. Entlang einer in dem in Fig. 2 im entfalteten Zustand gezeigten Höschen quer verlaufenden Mittellinie kann das Höschen in Längsrichtung übereinandergelegt werden, wobei sich die innere Lage 4 an der Innenseite befindet, und gegenüberliegende Bereiche 11 können anschließend mit Ausnahme der Beinöffnungen 3 miteinander verklebt werden, um so das in Fig. 1 isometrisch dargestellte Höschen zu bilden.

Die innere Lage 4 besteht aus geeigneten thermoplastischen gekräuselten Fasern, die mit einem bekannten Spritzdüsenfaserumschlingungsverfahren zu einem elastisch in Länge und Breite dehnbaren Vliesstoff geformt werden. Eine vorgegebene Zone der inneren Lage 4 kann mit einem bekannten, Hydrophilität verleihenden Mittel (wie z.B. einer grenzflächenaktiven Substanz) behandelt werden, um eine Feuchtigkeitsfühlzone 12 bilden, die durch flüssige Ausscheidungen in hohem Maß befeuchtbar ist. Alternativ kann das Vlies aus geeigneten hydrophilen Fasern, wie z.B. Rayonfasern, oder hydrophoben Synthetikfasern, denen hydrophile Eigenschaften verliehen wurden (wie z. B. Polyesterfasern, die hydrophil gemacht wurden ) oder einer Kombination dieser Fasern gebildet sein, oder es kann mit hydrophilen Eigenschaften versehenes Papier (Japanpapier) oder ähnliches in einer vorgegebenen Größe zugeschnitten werden und fest mit der Oberseite dieser bestimmten Zone verklebt werden. Auch ist es möglich, die gesamte innere Lage 4 als Feuchtigkeitsfühlzone 12 zu nutzen. In diesem Fall kann ein Vliesstoff aus hydrophilen Fasern oder aus Fasern, denen eine hydrophile Eigenschaft verliehen wurde, oder, falls erwünscht, aus einer Kombination derartiger Fasern mit hydrophoben Fasern verwendet werden.

Die äußere Lage 5 ist aus Vliesstoff gebildet, der aus dem selben Material wie die innere Lage 4 besteht, wobei dieser Vliesstoff flüssigkeitsdurchlässig und in Länge und Breite elastisch dehnbar ist, und eine in ähnlicher Weise elastisch in Länge und Breite dehnbare Folie aus synthetischem Harz oder Gummi ist fest mit dem Vliesstoff verklebt. Alternativ ist es möglich, elastisch in Länge und Breite dehnbaren Vliesstoff zu verwenden, der flüssigkeitsundurchlässig ist oder dem Durchtritt von Flüssigkeit widersteht.

Die vorliegende Erfindung betrifft Erziehungshöschen, und es ist daher nicht zwingend notwendig, in das Produkt den flüssigkeitsabsorbierenden Kern 6 einzugliedern. Wird, wie in der dargestellten Ausführungsform, ein derartiger Kern 6 verwendet, so ist es nicht wünschenswert, daß der Kern 6 eine ausreichend hohe Flüssigkeitsabsorptionskraft hat, um die die innere Lage 4 befeuchtenden flüssigen Ausscheidungen zu absorbieren und so das Feuchtigkeitsgefühl zu vermindern, das dem das Höschen tragenden Baby vermittelt werden soll.

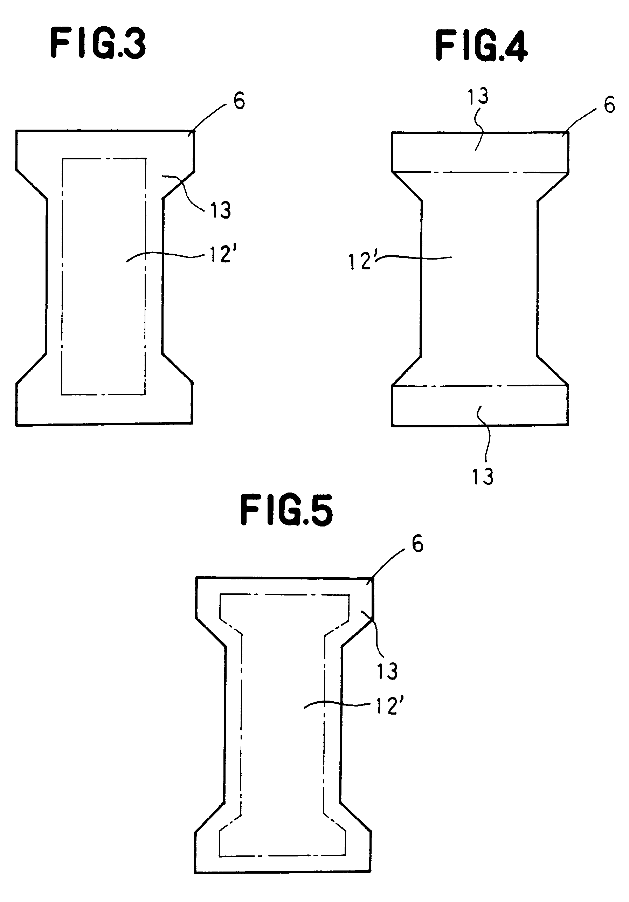

Wenn jedoch gewünscht wird, den Kern 6 zu verwenden, um des weiteren das Austreten von flüssigen Ausscheidungen zuverlässig zu vermeiden, wird der Kern 6 vorzugsweise so gewählt, daß die einmal absorbierten flüssigen Ausscheidungen wieder ohne weiteres abgegeben werden. Fig. 3 bis 5 zeigen verschiedene Beispiele, die jeweils anstelle der oder zusätzlich zur Feuchtigkeitsfühlzone 12 eine Feuchtigkeitsfühlzone 12' enthalten.

Wie in Fig. 3 bis 5 gezeigt, ist der Kern 6 in einem bestimmten Bereich mit einer Feuchtigkeitsfühlzone 12' versehen, die durch die unterbrochenen Linien angezeigt ist. Genauer heißt das, daß die Dichte des Kerns in der Feuchtigkeitsfühlzone 12' gegenüber der Dichte im übrigen Teil des Kerns verringert sein kann. Besteht der Kern 6 aus einer Mischung von Fasermasse mit hoch absorptivem Polymerpulver, so sollte die in der Feuchtigkeitsfühlzone 12' enthaltenen Menge von hoch absorptivem Polymerpulver geringer sein als die im Rest des Kerns enthaltene Menge, da mit geringerer Dichte oder verminderter Menge von hoch absorptivem Polymerpulver die Absorptionskraft für flüssige Ausscheidungen entsprechend verringert ist. Um die Dichte in einem begrenzten Gebiet des Kerns 6 zu verringern, kann die Materialmenge oder Stärke in dem Bereich, in dem keine Verringerung erforderlich ist, so eingestellt werden, daß sie größer ist als in der Zone, in der eine Verringerung erforderlich ist, worauf der gesamte Kern 6 auf eine im wesentlichen gleichmäßige Stärke zusammengepreßt werden kann.

Wenn sich ein das Höschen tragendes Baby hinsetzt, wird auf das Höschen eine Belastung von annähernd 35g/cm² ausgeübt. Entsprechend ist die Feuchtigkeitsfühlzone 12' vorzugsweise so zu konstruieren, daß die einmal in dieser absorbierten flüssigen Ausscheidungen unter einer Last von weniger als 35g/cm² austreten.

Die Feuchtigkeitsfühlzone 12' dient dazu, die an dieser anliegende Feuchtigkeitsfühlzone 12 erneut zu befeuchten und dadurch den Feuchtigkeitsfühleffekt zu verbessern.

## Patentansprüche

1. Wegwerfhöschen zur Erziehung, mit einer elastisch dehnbaren Hüftöffnung (2) und zwei elastisch dehnbaren Beinöffnungen (3), das eine flüssigkeitsdurchlässige innere Lage (4), eine flüssigkeitsundurchlässige äußere Lage (5) und einen zwischen dieser inneren und äußeren Lage liegenden absorbierenden Kern (6) umfaßt, **gekennzeichnet durch** eine vliesartige Lage (12), die an einem bestimmten Abschnitt der inneren Oberfläche der inneren Lage befestigt ist und die Feuchtigkeitsrückhalteeigenschaften aufweist, die größer sind als derartige Eigenschaften der inneren Lage, um eine Feuchtigkeitsfühlzone (Ffz) zu schaffen, die von dem Träger als unangenehm empfunden wird, sobald eine Ausscheidung stattgefunden hat.

2. Höschen nach Anspruch 1, wobei der Saugkern (6) mit einer Feuchtigkeitsfühlzone an der Stelle der Wahrnehmzone ausgestattet ist, und diese Feuchtigkeitsfühlzone unter einer vorbestimmten Last die Eigenschaft aufweist, mehr flüssige Ausscheidungen abzusondern als der Rest des Kerns.

3. Höschen nach Anspruch 2, wobei diese Last weniger als 35 g/cm2 beträgt.

## Claims

1. Disposable training pants, with an elastically expandable hip opening (2) and two elastically expandable leg openings (3), including a liquid-permeable inner layer (4), a liquid-impermeable outer layer (5) and an absorbent core (6) located between these inner and outer layers, characterised by a fleece-like layer (12) which is secured to a specific portion of the inner surface of the inner layer, and which has moisture-retaining properties which are greater than similar properties of the inner layer, in order to provide a moisture sensor zone (Ffz) which is perceived as unpleasant by the wearer as soon as a deposit has taken place.

2. Pants according to Claim 1, the absorbent core (6) being provided with a moisture sensor zone at the point of the awareness zone, and this moisture sensor zone, under a predetermined load, has the property of separating more liquid deposits than the remainder of the core.

3. Pants according to Claim 2, this load being less than 35 g/cm².

## Revendications

1. Culotte d'apprentissage de la propreté, comportant une ouverture de passage des hanches (2) élastique et étirable, et deux ouvertures de passage des jambes (3) élastiques et étirables, et qui comprend une couche intérieure perméable aux liquides (4), une couche extérieure imperméable aux liquides (5), et un corps central absorbant (6) intercalé entre ces couches intérieure et extérieure, caractérisée par une couche (12) de type non tissé, fixée à une portion déterminée de la surface intérieure de la couche intérieure, et présentant des propriétés de rétention des liquides, qui sont supérieures aux propriétés analogues de la couche intérieure, afin de créer une zone de sensation de l'humidité (Ffz), qui est perçue comme désagréable par le porteur dès qu'une excrémentation a eu lieu.

2. Culotte selon la revendication 1, dans laquelle le corps central absorbant (6) est muni d'une zone de sensation de l'humidité à l'emplacement de la zone perceptive, et dans laquelle cette zone de sensation de l'humidité a la propriété, sous une charge prédéterminée, de sécréter plus d'excréments liquides que le reste du corps central.

3. Culotte selon la revendication 2, dans laquelle cette charge est inférieure à 35 g/cm².
